# EUROPÄISCHE PATENTSCHRIFT

(11) **EP 1 007 105 B1**
(45) Veröffentlichungstag und Bekanntmachung des Hinweises auf die Patenterteilung: **26.03.2003**
(21) Anmeldenummer: 98945273.5
(22) Anmeldetag: 20.08.1998
(51) Int. Cl.: A61K 49/04

(54) **VERFAHREN ZUM HERSTELLEN VON NEBENWIRKUNGSFREIEN KONSTRASTMITTELN BEI ULTRAFILTRATION**
METHOD FOR PRODUCING CONTRAST AGENTS LARGELY FREE OF SIDE-EFFECTS
PROCEDE PERMETTANT DE FABRIQUER DES PRODUITS DE CONTRASTE PRATIQUEMENT DEPOURVUS D'EFFETS SECONDAIRES

(30) Priorität: 21.08.1997 DE 19736472
(43) Veröffentlichungstag der Anmeldung: 14.06.2000
(73) Patentinhaber: Amersham Health AS, 0401 Oslo (NO)
(72) Erfinder: Amersham Health AS, 0401 Oslo (NO)
(74) Vertreter: Rollins, Anthony John
(86) Internationale Anmeldenummer: EP9805305
(87) Internationale Veröffentlichungsnummer: WO99010015

(56) Entgegenhaltungen:
- EP-A- 0 312 104
- EP-A- 0 882 454
- WO-A-92/14539
- WO-A-94/14478
- WO-A-97/30735
- US-A- 5 204 005
- US-A- 5 550 287
- US-A- 5 779 905
- VON BARTHELD, C. S.: "Radio-iodination of neurotrophins and their delivery in vivo: advantages of membrane filtration and the use of disposable syringes" JOURNAL OF NEUROSCIENCE METHODS,1998, Seiten 207-215, XP002090454
- FABER, J. ET AL: "Serum free T4, T3, rT3, 3,3'-diiodothyronine and 3',5'-diiodothyronine measured by ultrafiltration" ACTA ENDOCRINOLOGICA,1984, XP002090356
- KAZIKIEWICZ J M ET AL: "RAPID MINIATURIZED CHROMATOGRAPHY PROCEDURES FOR IODINATED MONOCLONAL ANTIBODIES COMPARISON TO GEL EXCLUSION CHROMATOGRAPHY" J NUCL MED TECHNOL, XP002090126

## Beschreibung

Die Erfindung bezieht sich auf ein Verfahren zum Herstellen von weitgehend nebenwirkungstreien Kontrastmitteln, insbesondere nichtionischen, monomeren oder dimeren Röntgenkontrastmitteln, bei dem das Kontrastmittel in Kontakt mit einem reaktiven Medium mit gegenüber dem Kontrastmittel größeren Molekülen gebracht wird, so daß im Kontrastmittel herstellungsbedingt enthaltene reaktive Verunreinigungen mit dem reaktiven Medium abreagieren, und daß die sich durch ihre Molekülgröße vom Kontrastmittel unterscheidenden Reaktionsprodukte vom Kontrastmittel abgetrennt werden.

Derartige Verfahren, bei denen Kontrastmittel mit Aktivkohle als reaktives Medium oder mit Hilfe von Ionenaustauschern gereinigt wird, ist bekannt.

Kontrastmittel werden gezielt in den menschlichen Körper eingeführt, beispielsweise durch Einspritzen in die Blutbahn, um bestimmte Körperpartien oder auch die Strömung des Blutes mittels Röntgenstrahlen sichtbar zu machen, die beim Durchleuchten vom Kontrastmittel besonders stark geschwächt werden. Nach Ausübung dieser Funktion verbleibt das Kontrastmittel im Körper und wird allmählich über die entsprechenden Organe wieder ausgeschieden. Es kommt also zu einem intensiven Körperkontakt mit der Gefahr von unerwünschten Nebenwirkungen. So wurden beim Menschen allergische und pseudoallergische Reaktionen, die spontan oder verzögert auftreten können, als Folge von Kontrastmittelgaben beobachtet. Diese Reaktionen lassen sich auch mit der bekannten Kontrastmittelreinigung nicht wesentlich verringern.

Früher wurden als Kontrastmittel ionische Substanzen (Salze) wie Jodide eingesetzt, die pro wirksames Jodmolekül zwei osmotisch wirksame Teilchen aufweisen. Inzwischen sind nichtionische monomere Kontrastmittel auf dem Markt, die drei Jodatome kovalent an einem Benzolring als Grundgerüst enthalten und deren Wasserlöslichkeit durch mehrere OH-Gruppen an Seitenketten erzielt wird. Durch diese Anordnung sind pro osmotisch wirksames Teilchen drei Jodmoleküle vorhanden. Die neueste Entwicklung, die sogenannten dimeren nichtionischen Kontrastmittel, entstehen im Prinzip durch Verknüpfung von zwei nichtionischen monomeren Grundgerüsten und verdoppeln somit die Anzahl der Jodatome pro osmotisch wirksames Teilchen auf sechs. Obwohl die Nebenwirkungen bei den nichtionischen gegenüber den ionischen Kontrastmitteln deutlich abgenommen haben, muß man bei der Verabreichung dieser Substanzen immer noch mit Nebenwirkungen und Spätreaktionen allergischer und pseudoallergischer Natur rechnen, die in seltenen Fällen bis zum Exitus des Patienten führen können. Bei den nichtionischen dimeren Kontrastmitteln im speziellen ist ein Trend zu pseudoallorgischen Spätreaktionen beobachtet worden. Eine Reduzierung von Nebenwirkungen bei allen Röntgenkontrastmitteln wäre daher von medizinischer Relevanz.

Für die vorliegende Erfindung ist bedeutsam, daß allergische Reaktionen nicht direkt vom Kontrastmittel ausgelöst werden, da dessen Moleküle viel zu klein sind, um vom Körper als Allergen erkannt zu werden. Die allergene Wirkung kommt daher erst zum Tragen, wenn sich ein Kontrastmittelmolekül an ein größeres Molekül gebunden hat, beispielsweise an ein Albuminmolekül. Pseudoallergische Reaktionen, deren Auslöser und Mechanismus heute noch ungeklärt sind, werden ebenfalls wahrscheinlicher von einer kleinen Menge an reaktiver Verunreinigung(en) ausgelöst als vom Kontrastmittel selbst. Würde das Kontrastmittel diese Reaktionen verursachen, sollten Mengen von bis zu 300 ml und mehr zu weitaus höheren Nebenwirkungsraten führen. Man sollte sich vor Augen halten, daß das Injizieren von 300 ml eines handelsüblichen Kontrastmittels mit der Reinheit von 99,5% bedeutet, gleichzeitig eine Menge von 1,5 ml an unbekannten Verunreinigungen zu applizieren. Deswegen kann davon ausgegangen werden, daß es sich bei den die unerwünschten Reaktionen auslösenden Substanzen nicht um das Kontrastmittel selbst sondern um darin vorhandene geringe Spuren von Verunreinigungen handelt, die bei der Synthese des Kontrastmittels anfallen und auch bei sorgfältiger Reinigung in bekannter Weise nicht restlos entfernt werden können.

Hier setzt die Erfindung ein. Sie beruht auf dem Gedanken, die bisher im Körper stattfindende Reaktion vorweg außerhalb des Körpers durchzuführen und die dabei entstehenden Reaktionsprodukte vom Kontrastmittel abzutrennen, bevor dieses zur Anwendung kommt. Das bedeutet eine Reinigung des Kontrastmittels durch Vorinkubation.

Dementsprechend ist das erfindungsgemäße Verfahren dadurch gekennzeichnet, daß als reaktives Medium Substanzen menschlichen, tierischen oder pflanzlichen Ursprungs eingesetzt werden.

Somit wird erfindungsgemäß eine in vitro Vorinkubation durchgeführt, die zu einer deutlich erhöhten Verträglichkeit des so behandelten und separierten Kontrastmittels führt.

Zweckmäßige Maßnahmen bei der Durchführung des erfindungsgemäßen Verfahrens sind in den Unteransprüchen aufgeführt. Danach können als reaktive Substanzen menschlichen, tierischen oder pflanzlichen Ursprungs Proteine oder Albumine und insbesondere Blutproteine oder Blut, namentlich auch Humanblut, eingesetzt werden, was die bisher bei Kontrastmittelgaben auftretenden Verhältnisse und Vorgänge im Körper in besonderem Maße vorwegnimmt.

Beispiel: Nach der Synthese wird das Kontrastmittel mit einem reaktiven Medium in Kontakt gebracht, um die reaktiven Verunreinigungen abreagieren zu lassen. Dabei wird als reaktives Medium humanes Vollblut verwendet, um die Bedingungen im Körper möglichst identisch nachzuvollziehen. Das reaktive Medium wird bei einer erhöhten Temperatur von beispielsweise 37°C mit dem Kontrastmittel inkubiert. Anschließend wird das entstehende Reaktionsprodukt einschließlich des nicht verbrauchten reaktiven Mediums abgetrennt, was kostengünstig einfach durch Ultrafiltration erfolgt, die gleichzeitig mit dem letzten Produktionsschritt durchgeführt werden kann.

Alternativ zur Ultrafiltration kann bei einem unlöslichen reaktiven Medium das Kontrastmittel einfach durch eine Granulatschicht aus dem reaktiven Medium geleitet werden. Ähnlich kann mit anderen reaktiven Medien gearbeitet werden, wenn diese an einem inerten Trägermaterial gebunden sind.

Obwohl humanes Vollblut alle möglichen Reaktionspartner enthält, mit denen das Kontrastmittel bei Verabreichung im Körper in Berührung kommt, und außerdem alle medizinisch bedenklichen Komponenten von Humanblut durch die Ultrafiltration bzw. die Abtrennung des erfindungsgemäß behandelten Kontrastmittels wieder entfernt werden, ist es sowohl aus medizinischer wie auch aus finanzieller Sicht von Vorteil, den tatsächlichen Reaktionspartner im Blut zu identifizieren und in gereinigter Form für die Vorinkubation einzusetzen. Dabei kann wie folgt vorgegangen werden:

Durch Inkubation von radioaktiv markiertem Röntgenkontrastmittel mit Vollblut und anschließendes mehrfaches Auswaschen mittels Ultrafiltration wird ein Protein-Rückstand gewonnen, der analysiert wird, wobei das nun radioaktive Produkt elektrophoretisch identifiziert wird und dadurch der Reaktionspartner bestimmt wird, mit dem das Kontrastmittel gezielt gereinigt werden kann.

Das erfindungsgemäße Verfahren beschränkt sich nicht auf die vorstehend in erster Linie angesprochenen Röntgenkontrastmittel, vielmehr lassen sich auch andere Kontrastmittel wie beispielsweise Magnetresonanz-Kontrastmittel in der beschriebenen Weise in vitro vorinkubieren und reinigen und dadurch hinsichtlich ihrer Verträglichkeit verbessern. Im übrigen sind dem Fachmann in Betracht kommende Kontrastmittel bekannt - beispielsweise aus den Angaben in WO 97/30 735.

## Patentansprüche

1. Verfahren zum Herstellen von weitgehend nebenwirkungsfreien Kontrastmitteln umfassend
a) Inkontaktbringen des Kontrastmittels mit einem reaktiven Medium mit gegenüber dem Kontrastmittel grösseren Molekülen und Abreaktion der im Kontrastmittel herstellungsbedingt enthaltenen reaktiven Verunreinigungen mit dem reaktiven Medium zu Reaktionsprodukten, welche sich durch ihre Molekülgrösse vom Kontrastmittel unterscheiden und
b) Abtrennen der Reaktionsprodukte,
**dadurch gekennzeichnet, dass** als reaktives Medium menschliches oder tierisches Blut oder menschliche oder tierische Blutproteine eingesetzt werden.

2. Verfahren nach Anspruch 1, **dadurch gekennzeichnet, dass** als reaktives Medium Albumine eingesetzt werden

3. Verfahren nach Anspruch 1 und 2, **dadurch gekennzeichnet, dass** die Abreaktion bei erhöhter Temperatur durchgeführt wird.

4. Verfahren nach Anspruch 3, **dadurch gekennzeichnet, dass** die Temperatur 35 bis 40 °C beträgt.

5. Verfahren nach Anspruch 1 bis 4, **dadurch gekennzeichnet, dass** die Reaktionsprodukte durch Ultrafiltration abgetrennt werden.

6. Verfahren nach Anspruch 1 bis 5, **dadurch gekennzeichnet, dass** das reaktive Medium an einen inerten Träger gebunden ist

7. Verfahren nach Anspruch 1 bis 6, **dadurch gekennzeichnet, dass** es sich bei dem Kontrastmittel um ein Röntgenkontrastmittel oder ein Magnetresonanz-Kontrastmittel handelt.

8. Verfahren nach Anspruch 1 bis 7, **dadurch gekennzeichnet, dass** es sich bei dem Kontrastmittel um ein nichtionisches, monomeres oder dimeres Röntgenkontrastmittel handelt.

## Claims

1. A method for producing contrast agents largely free of side effects comprising:
a) contacting a contrast agent with a reactive medium, said reactive medium having molecules larger than in the contrast agent and reaction of the reactive contaminants contained in the contrast agent with the reactive medium to form reaction products which differ from the contrast agent in their molecular size and
b) separating off the reaction products
**characterised in that** human or animal blood or human or animal blood proteins are used as the reactive medium.

2. Method according to claim 1, **characterised in that** albumines are used as the reactive medium

3. Method according to claims 1 and 2, **characterised in that** the reaction is carried out at elevated temperature

4. Method according to claim 3, **characterised in that** the temperature is 35 to 40 °C.

5. Method according to claims 1 to 4, **characterised in that** the reaction products are separated off by ultrafiltration.

6. Method according to claims 1 to 5, **characterised in that** the reactive medium is bound to an inert support.

7. Method according to claims 1 to 6, **characterised in that** the contrast agent is a X-ray contrast agent or a magnetic resonance contrast agent.

8. Method according to claims 1 to 7, **characterised in that** the contrast agent is a non-ionic, monomeric or dimeric X-ray contrast agent.

## Revendications

1. Procédé pour préparer des produits de contraste largement dépourvus d'effets secondaires, comprenant :
a) la mise en contact du produit de contraste avec un milieu réactif contenant des molécules plus grosses que le produit de contraste, et réaction des impuretés réactives résultant du procédé de production, contenues dans le produit de contraste, avec le milieu réactif pour donner des produits de réaction qui diffèrent, par la taille de leurs molécules, du produit de contraste, et
b) la séparation des produits de réaction,
**caractérisé en ce qu'**on utilise comme milieu réactif du sang humain ou animal ou des protéines sanguines humaines ou animales.

2. Procédé selon la revendication 1, **caractérisé en ce qu'**on utilise comme milieu réactif des albumines.

3. Procédé selon la revendication 1 et 2, **caractérisé en ce que** la réaction est conduite à une température élevée.

4. Procédé selon la revendication 3, **caractérisé en ce que** la température est comprise entre 35 et 40 °C.

5. Procédé selon les revendications 1 à 4, **caractérisé en ce que** les produits de réaction sont séparés par ultrafiltration.

6. Procédé selon les revendications 1 à 5, **caractérisé en ce que** le milieu réactif est lié à un support inerte.

7. Procédé selon les revendications 1 à 6, **caractérisé en ce que** le produit de contraste est un produit de contraste pour radiographie ou un moyen de contraste pour résonance magnétique.

8. Procédé selon les revendications 1 à 7, **caractérisé en ce que** le produit de contraste est un produit de contraste pour radiographie non ionique, monomère ou dimère.
